# EUROPÄISCHE PATENTSCHRIFT

(11) **EP 2 303 347 B1**
(45) Veröffentlichungstag und Bekanntmachung des Hinweises auf die Patenterteilung: **20.03.2013**
(21) Anmeldenummer: 09777583.7
(22) Anmeldetag: 21.07.2009
(51) Int. Cl.: A61L 29/16

(54) **VERFAHREN ZUR ERZEUGUNG EINER BIOAKTIVEN OBERFLÄCHE AUF DEM BALLON EINES BALLONKATHETERS**
METHOD FOR PRODUCING A BIOACTIVE SURFACE ON THE BALLOON OF A BALLOON CATHETER
PROCÉDÉ DE PRODUCTION D'UNE SURFACE BIOACTIVE SUR LE BALLONNET D'UN CATHÉTER À BALLONNET

(30) Priorität: 22.07.2008 DE 102008034826
(43) Veröffentlichungstag der Anmeldung: 06.04.2011
(73) Patentinhaber: Rübben, Alexander, 98000 Monaco (MC)
(72) Erfinder: Rübben, Alexander, 98000 Monaco (MC)
(74) Vertreter: Schöneborn, Holger
(86) Internationale Anmeldenummer: PCT/EP2009/005568
(87) Internationale Veröffentlichungsnummer: WO 2010/009904

(56) Entgegenhaltungen:
- WO-A2-2008/061642
- US-A1- 2007 088 255
- US-B1- 6 287 628
- US-B1- 6 369 039

## Beschreibung

Die vorliegende Erfindung betrifft ein Verfahren zur Erzeugung einer bioaktiven Oberfläche auf dem Ballon eines Ballonkatheters. Die Erfindung betrifft zudem einen Ballon eines Ballonkatheters und einen Ballonkatheter.

Die sogenannten "minimalinvasiven Verfahren" nehmen in der Medizin einen immer größeren Stellenwert ein. Im Rahmen der Radiologie ist hierbei die interventionelle Radiologie anzusprechen, die wesentlich zur Entwicklung minimalinvasiver Techniken und hierfür notwendiger Geräte und Prothesen aus geeigneten Materialien beigetragen hat. So werden heute Ballonkatheter sowohl von Kardiologen als auch von Radiologen in Gefäße eingesetzt, um diese zu öffnen. Bei diesen Eingriffen kann es zu einer Verdickung der Gefäßwand mit konsekutiver Lumeneinengung im Bereich der Aufdehnung durch eine Zellproliferation kommen.

Durch Medikamentenabgabe von der Oberfläche des Ballons des Ballonkatheters kann diesem Problem entgegengewirkt werden. Typischerweise wird ein in einem Lösungsmittel gelöster Wirkstoff auf die Oberfläche des Ballons des Ballonkatheters aufgebracht, wobei das Lösungsmittel anschließend verdunstet. Der Wirkstoff befindet sich dann als Schicht auf der Oberfläche.

Möglichkeiten zur Erzielung einer demgegenüber verbesserten Haftung des Wirkstoffes auf der Oberfläche werden beispielsweise in den Dokumenten US 5,102,402 und US 6,129,705 beschrieben. In dem Dokument US 5,102,402 ist ein mit Medikamenten beschichteter Ballonkatheter beschrieben. Dabei werden in einer ersten Variante mit einem Wirkstoff bzw. Medikament gefüllte Mikrokapseln von Falten in der Ballonoberfläche umschlossen und so mechanisch in der jeweiligen Position gehalten. In einer zweiten Variante sind die Mikrokapseln mit Hilfe eines Haftvermittlers auf die Ballonoberfläche geklebt.

In dem Dokument US 6,129,705 wird ein Ballonkatheter beschrieben, dessen Oberfläche eine Beschichtung aufweist, in die mit einem Wirkstoff gefüllte Mikrokapseln vollständig eingebettet sind. Allerdings handelt es sich bei dem Einfüllen des Wirkstoffes in Mikrokapseln und dem anschließenden Befestigen bzw. Einbetten der Mikrokapseln auf der Ballonoberffäche um vergleichsweise aufwendige und damit kostenintensive Verfahren.

Grundsätzlich ist es wünschenswert, wenn die Oberfläche des Ballons des Ballonkatheters eine homogene und reproduzierbare Medikamentenbeladung aufweist und sich gleichzeitig durch eine gleichmäßige Medikamentenabgabe an das umliegende Gewebe im Körper auszeichnet.

Es ist daher eine erste Aufgabe der vorliegenden Erfindung, ein vorteilhaftes Verfahren zur Erzeugung einer bioaktiven Oberfläche auf dem Ballon eines Ballonkatheters zur Verfügung zu stellen. Es ist eine zweite Aufgabe der Erfindung, einen vorteilhaften Ballon eines Ballonkatheters zur Verfügung zu stellen. Eine dritte Aufgabe besteht darin, einen vorteilhaften Ballonkatheter zur Verfügung zu stellen.

Die erste Aufgabe wird durch ein Verfahren nach Anspruch 1, die zweite Aufgabe durch einen Ballon eines Ballonkatheters nach Anspruch 13 und die dritte Aufgabe durch einen Ballonkatheter nach Anspruch 14 gelöst. Die abhängigen Ansprüche enthalten weitere vorteilhafte Ausgestaltungen der Erfindung. Die Merkmale sind sowohl einzeln als auch in Kombination miteinander vorteilhaft.

In dem erfindungsgemäßen Verfahren zur Erzeugung einer bioaktiven Oberfläche auf dem Ballon eines Ballonkatheters wird die Oberfläche des Ballons zumindest teilweise mit einer ersten Lösung eines Wirkstoffes benetzt. Anschließend wird der mit der ersten Lösung eines Wirkstoffes benetzte Teil der Oberfläche des Ballons mit einer zweiten, gesättigten Lösung des Wirkstoffes benetzt. Dabei sollte die mit der ersten Lösung eines Wirkstoffes benetzte Oberfläche des Ballons langsam trocknen.

Durch das Benetzen der Oberfläche des Ballons mit der ersten Lösung eines Wirkstoffes wird auf der Oberfläche eine lackartige, transparente Wirkstoffschicht erzeugt, die als Basis für eine homogene und reproduzierbare Wirkstoffbeladung dient. Dabei wird ein großer beziehungsweise der größte Anteil an der gesamten Wirkstoffbeladung aufgebracht. Während des Benetzens kann das Ballonmaterial, insbesondere im Fall der Verwendung von Methylenchlorid Lösungsmittel aufnehmen, das unter Normalbedingungen innerhalb weniger Stunden wieder abgegeben wird.

Durch das Benetzen mit der zweiten gesättigten Lösung eines Wirkstoffes wird die gesamte Beschichtung spröder und optisch weniger transparent, also milchiger. Die so erzeugte Oberfläche hat eine kreideartige, insbesondere nicht-kristalline, Konsistenz, die einen erhöhten Wirkstoffabtrag bei Reibung ermöglicht als im Falle einer Beschichtung nur durch Benetzen der Oberfläche des Ballons mit der ersten Lösung des Wirkstoffes. Durch das erfindungsgemäße Verfahren wird eine höhere Wirkstoffabgabe vom Ballon an das umliegende Gewebe im Körper erreicht als im Falle einer Beschichtung nur durch Benetzen der Oberfläche des Ballons mit der ersten Lösung des Wirkstoffes.

Grundsätzlich kann sowohl die gesamte Ballonoberfläche, als auch nur ein Teil der Ballonoberfläche, beispielsweise der beim Aufdehnen mit dem Gewebe in Kontakt kommende Bereich der Oberfläche, mit dem erfindungsgemäßen Verfahren beschichtet werden. Insbesondere kann der Ballon einen zylinderförmigen Bereich und mindestens einen konusförmigen Bereich umfassen. In diesem Fall kann beispielsweise nur der zylinderförmige Bereich des Ballons mit einem Wirkstoff erfindungsgemäß beschichtet werden oder der zylinderförmige Bereich des Ballons und ein konusförmiger Bereich.

Vorzugsweise kann die zweite, gesättigte Lösung Ethanol oder Azeton umfassen. In Ethanol oder Azeton ist der verwendete Wirkstoff weniger löslich als in dem im Rahmen der ersten Lösung verwendeten Lösungsmittel, In Folge dessen wird während des Benetzens mit der zweiten, gesättigten Lösung ein Teil des Wirkstoffes aus der im Rahmen der Behandlung mit der ersten Lösung erzeugten Beschichtung herausgelöst beziehungsweise diese Beschichtung wird durch die zweite Lösung angegriffen und die Oberfläche wird poröser beziehungsweise versprödet partiell.

Weiterhin kann die zweite, gesättigte Lösung ein azeotropes Lösungsmittel umfassen.

Als erste Lösung kann insbesondere eine gesättigte Lösung verwendet werden, vorzugsweise eine gesättigte Lösung des Wirkstoffes Paclitaxel in Methylenchlorid. Andere Lösungsmittel, wie beispielsweise Chloroform oder Ethanol, oder Lösungsmittelgemische können ebenfalls verwendet werden.

Vorteilhafterweise kann die Oberfläche des Ballons mit der ersten Lösung eines Wirkstoffes durch Eintauchen des Ballons in die erste Lösung benetzt werden. Der Ballon kann nach dem Eintauchen mit einer Geschwindigkeit von bis zu 10 mm/s aus der ersten Lösung herausgezogen werden. Noch günstiger ist es, wenn das Herausziehen mit einer Geschwindigkeit von weniger als 5 mm/s, vorzugsweise mit einer Geschwindigkeit zwischen 0,5 mm/s und 2 mm/s, erfolgt. Durch das langsame Herausziehen wird ein langsames Trocknen der Oberfläche und damit die Ausbildung einer lackartigen Wirkstoffschicht erreicht.

Zudem kann die Oberfläche des Ballons vor dem Benetzen mit der ersten Lösung eines Wirkstoffes gereinigt und/oder mit einer Strukturierung beziehungsweise Profilierung versehen werden. Die Oberfläche des Ballons kann beispielsweise mechanisch, thermisch oder chemisch strukturiert beziehungsweise profiliert werden. Insbesondere kann die Oberfläche durch Aufrauen strukturiert beziehungsweise profiliert werden. Vorteilhafterweise werden durch das Vergrößern der Oberfläche des Ballons auf der Oberfläche Vertiefungen mit einer Tiefe von 5-50 µm und einer Breite von 5-50 µm erzeugt.

Weiterhin kann die Oberfläche des Ballons nach dem Benetzen mit der ersten Lösung eines Wirkstoffes und vor dem Benetzen mit der zweiten, gesättigten Lösung mit einer weiteren, vorzugsweise nicht-gesättigten, Lösung des Wirkstoffes benetzt werden. Dadurch wird die Wirkstoffbeiadung erhöht. Die gesamte Beschichtung kann durch das Benetzen mit der weiteren Lösung des Wirkstoffes spröder werden beziehungsweise partiell verspröden. Zudem kann die gesamte Beschichtung optisch weniger transparent, also milchiger werden. Insgesamt bewirkt das Benetzen mit der weiteren Lösung des Wirkstoffes eine höhere Wirkstoffabgabe bei Reibung im Vergleich zu der durch das Benetzen mit der ersten Lösung entstandenen lackartigen Oberfläche.

Wird das Benetzen mit der weiteren Lösung eines Wirkstoffes nach dem Benetzen mit der ersten Lösung eines Wirkstoffes weggelassen, so ist im Ergebnis die Wirkstoffbeladung auf der Ballonoberfläche geringer.

Bei der weiteren Lösung kann es sich zum Beispiel um eine Lösung von Paclitaxel in Methylenchlorid handeln. Vorteilhafterweise sollte die Konzentration der Lösung geringer sein als die der ersten Lösung, beispielsweise 100 mg/ml. Andere Lösungsmittel, wie beispielsweise Chloroform oder Ethanol, oder Lösungsmittelgemische können ebenfalls verwendet werden.

Darüber hinaus kann die Oberfläche des Ballons mit der weiteren Lösung des Wirkstoffes durch Eintauchen des Ballons in die weitere Lösung benetzt werden. Die Oberfläche des Ballons kann insbesondere maximal 20 s lang, vorteilhafterweise zwischen 0,5 s und 10 s, in die weitere Lösung des Wirkstoffes eingetaucht beziehungsweise mit dieser benetzt werden. Die Dauer des Benetzens beziehungsweise des Eintauchens kann von der Wirkstoffbeladung nach dem Benetzen mit der ersten Lösung abhängig gemacht werden. Je höher die Wirkstoffbeladung nach dem Benetzen mit der ersten Lösung ist, desto länger sollte die Oberfläche des Ballons in die weitere Lösung des Wirkstoffes eingetaucht beziehungsweise mit dieser benetzt werden. Vorzugsweise kann die Oberfläche des Ballons mit der nicht-gesättigten Lösung des Wirkstoffes maximal 3 min nach dem Benetzen mit der ersten Lösung des Wirkstoffes benetzt werden.

Weiterhin kann das Benetzen der Oberfläche des Ballons mit der zweiten, gesättigten Lösung des Wirkstoffes durch Eintauchen des Ballons in die zweite, gesättigte Lösung erfolgen. Die Oberfläche des Ballons kann dabei insbesondere maximal 20 s lang, vorteilhafterweise zwischen 0,5 s und 10 s, in die zweite, gesättigte Lösung des Wirkstoffes eingetaucht beziehungsweise mit dieser benetzt werden. Die Dauer des Benetzens beziehungsweise des Eintauchens kann von der Wirkstoffbeladung nach dem Benetzen mit der ersten Lösung beziehungsweise von der Wirkstoffbeladung nach dem Benetzen mit der weiteren Lösung abhängig gemacht werden. Je höher die Wirkstoffbeladung nach dem Benetzen mit der ersten Lösung beziehungsweise der weiteren Lösung ist, desto länger sollte die Oberfläche des Ballons in die zweite, gesättigte Lösung des Wirkstoffes eingetaucht beziehungsweise mit dieser benetzt werden. Vorzugsweise kann die Oberfläche des Ballons mit der zweiten, gesättigten Lösung des Wirkstoffes maximal 3 min nach dem Benetzen mit der ersten Lösung des Wirkstoffes beziehungsweise maximal 3 min nach dem Benetzen mit der weiteren Lösung benetzt werden.

Die Oberfläche des Ballons kann zudem nach dem Benetzen mit der weiteren Lösung des Wirkstoffes und/oder nach dem Benetzen mit der zweiten, gesättigten Lösung des Wirkstoffes getrocknet werden. Zum Beispiel kann der Ballon eine Längsachse umfassen und während des Trocknens um seine Längsachse gedreht werden. Zur möglichst gleichmäßigen Trocknung kann die Längsachse des Ballons unmittelbar nach dem Benetzen mit der weiteren Lösung und/oder nach dem Benetzen mit der zweiten, gesättigten Lösung in eine horizontale Lage gebracht werden. Der Ballon kann dann in einem Luftstrom um seine Längsachse gedreht werden.

Der verwendete Wirkstoff kann insbesondere Tretinoin und/oder Tretinoinderivate und/oder Orphanrezeptoragonisten und/oder Elafinderivate und/oder Corticosteroide und/oder Steroidhormone und/oder Paclitaxel und/oder Paclitaxelderivate und/oder Rapamune und/oder Tacrolimus und/oder hydrophobe Proteine und/oder zellproliferationsverändernde Substanzen oder Gemische daraus umfassen. Als Steroidhormone können beispielsweise Methylprednisolon, Dexamethason oder Östradiol verwendet werden.

Im Zusammenhang mit der ersten Lösung und/oder der zweiten, gesättigten Lösung und/oder der nicht-gesättigten Lösung können als Lösungsmittel beispielsweise Methylenchlorid, Chloroform, Alkohol, insbesondere Ethanol, Methanol oder Isopropanol, Azeton, Diethylether, flüssige Kohlenwasserstoffe, wie zum Beispiel Pentan, Hexan, Heptan, Cyklohexan oder Oktan, Toluol, Tetrahydrofuran (THF) oder, Essigester verwendet werden. Das verwendete Lösungsmittel kann insbesondere Ethanol und Wasser umfassen. Die zweite, gesättigte Lösung Essigsäure kann zudem umfassen.

Im Rahmen der zweiten, gesättigten Lösung können Lösungmittelgemische wie zum Beispiel ein Gemisch, welches Ethanol und Wasser umfasst, verwendet werden. Beispielsweise kann das azeotrope Lösungsmittelgemisch zu 96 % aus Ethanol und zu 4 % aus Wasser bestehen, bezogen jeweils auf das Gewicht. Dieses Lösungsmittelgemisch kann weiterhin mit 0,1 % Essigsäure, bezogen auf das Volumen, versetzt sein. Die Essigsäure bewirkt eine Stabilisierung des Wirkstoffes, beispielsweise des Paclitaxels.

Der erfindungsgemäße Ballon eines Ballonkatheters umfasst eine Oberfläche, die zumindest teilweise eine Beschichtung mit einem Wirkstoff aufweist. Die Beschichtung ist in dem gesamten beschichteten Bereich homogen und spröde ausgestaltet. Die Oberfläche kann insbesondere eine kreideartige beziehungsweise nicht-kristalline Struktur besitzen. Zudem kann die Oberfläche des Ballons sowohl vollständig als auch nur teilweise beschichtet sein. Insbesondere kann der Ballon einen zylinderförmigen Bereich und mindestens einen konusförmigen Bereich umfassen. In diesem Fall können beispielsweise nur der zylinderförmige Bereich des Ballons oder der zylinderförmige Bereich und ein konusförmiger Bereich mit einem Wirkstoff erfindungsgemäß beschichtet sein. Der erfindungsgemäße Ballon lässt sich grundsätzlich mit Hilfe des erfindungsgemäßen Verfahren herstellen. Er gewährleistet eine homogene und hohe Medikamentenabgabe an das umliegende Gewebe im Körper.

Der erfindungsgemäße Ballonkatheter umfasst einen zuvor beschriebenen erfindungsgemäßen Ballon und hat dieselben Vorteile wie der erfindungsgemäße Ballon.

Weitere Merkmale, Eigenschaften und Vorteile der vorliegenden Erfindung werden nachfolgend anhand eines Ausführungsbeispiels unter Bezugnahme auf die beiliegenden Figuren beschrieben. Die beschriebenen Merkmale sind sowohl einzeln als auch in Kombination miteinander vorteilhaft.
Fig. 1 zeigt schematisch einen Ballonkatheter.
Fig. 2 zeigt schematisch das Eintauchen des Ballonkatheters in die erste Lösung eines Wirkstoffes.
Fig. 3 zeigt schematisch einen Schnitt durch einen Teil des Ballons des Ballonkatheters nach dem Eintauchen in die erste Lösung.
Fig. 4 zeigt schematisch das Eintauchen des Ballonkatheters in die weitere, nicht-gesättigte Lösung.
Fig. 5 zeigt schematisch das Drehen des Ballonkatheters während des Trocknungsprozesses nach dem Eintauchen in die weitere, nicht-gesättigte Lösung bzw. in die zweite, gesättigte Lösung.
Fig. 6 zeigt schematisch einen Schnitt durch einen Teil der Oberfläche des Ballons des Ballonkatheters nach dem Eintauchen in die weitere, nicht-gesättigte Lösung.
Fig. 7 zeigt schematisch das Eintauchen des Ballonkatheters in die zweite, gesättigte Lösung.
Fig. 8 zeigt schematisch einen Schnitt durch einen Teil der Oberfläche des Ballons des Ballonkatheters nach dem Eintauchen in die zweite, gesättigte Lösung.

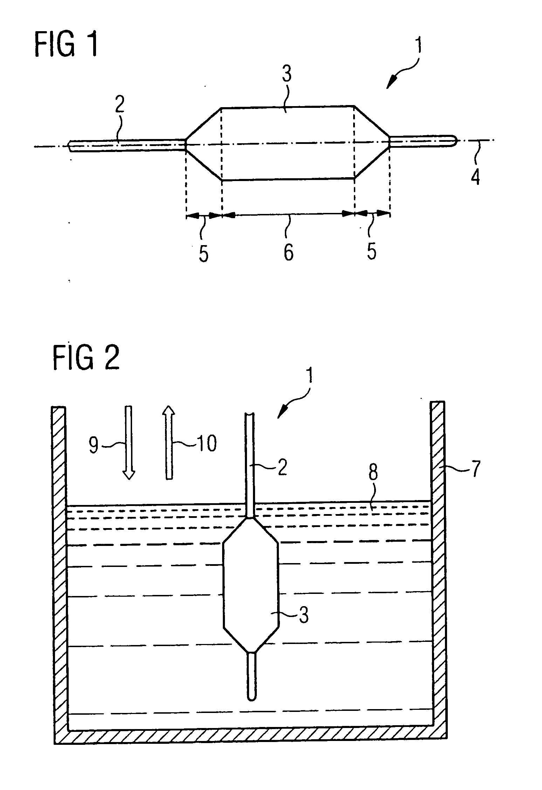

Im Folgenden wird ein Ausführungsbeispiel der vorliegenden Erfindung anhand der Figuren 1 bis 8 näher erläutert. Die Figur 1 zeigt schematisch einen Ballonkatheter 1. Der Ballonkatheter 1 umfasst eine Kathetersonde 2 und einen Ballon 3. Der Ballon 3 umschließt einen Teil der Kathetersonde 2. Die Längsachse des Ballonkatheters 1 ist durch die Bezugsziffer 4 gekennzeichnet. Der Ballon 3 umfasst zwei konusförmige Bereiche 5 und einen zwischen den beiden konusförmigen Bereichen 5 angeordneten zylinderförmigen Bereich 6.

Im Rahmen des erfindungsgemäßen Verfahrens wird der Ballon 3 des Ballonkatheters 1 zunächst in eine Lösung, vorzugsweise gesättigte Lösung, von beispielsweise Paclitaxel in Methylenchlorid eingetaucht. Dies ist schematisch in der Fig. 2 dargestellt. Die Figur 2 zeigt schematisch ein Gefäß 7 in welchem sich eine erste Lösung 8 befindet. Bei der ersten Lösung 8 handelt es sich z. B. um eine gesättigte Lösung von Paclitaxel in Methylenchlorid. Grundsätzlich kommen auch andere Lösungsmittel wie beispielsweise Chloroform, Ethanol oder Lösungsmittelgemische in Betracht. Der Ballon 3 kann vor dem Eintauchen in die erste Lösung 8 zunächst gereinigt beziehungsweise angeraut und gereinigt werden. Der Ballon 3 wird vorzugsweise senkrecht vollständig in die erste Lösung 8 eingetaucht. Das Eintauchen ist durch einen Pfeil 9 in der Figur 2 gekennzeichnet. Danach wird der Ballon 3 langsam, vorzugsweise mit einer Geschwindigkeit von ca. 1 mm/s, senkrecht aus der ersten Lösung 8 wieder herausgezogen. Das Herausziehen ist in der Figur 2 durch einen Pfeil 10 gekennzeichnet. Nach dem Herausziehen 10 wird der Ballon 3 langsam getrocknet.

Durch das Eintauchen in die erste Lösung 8 und das langsame Herausziehen und Trocknen des Ballons 3 entsteht auf dem Ballon 3 eine transparente, lackartige Paclitaxelschicht, die als Basis für eine homogene und reproduzierbare Gesamtbeladung des Ballons 3 mit dem Wirkstoff Paclitaxel dient. Durch das Eintauchen und langsame Herausziehen und Trocknen des Ballons 3 in die erste Lösung 8 wird ein großer beziehungsweise der größte Anteil des Wirkstoffes auf den Ballon 3 aufgebracht. Das Ballonmaterial nimmt während des Tauchprozesses deutlich Lösungsmittel auf, welches unter Normalbedingungen innerhalb weniger Stunden wieder abgegeben wird.

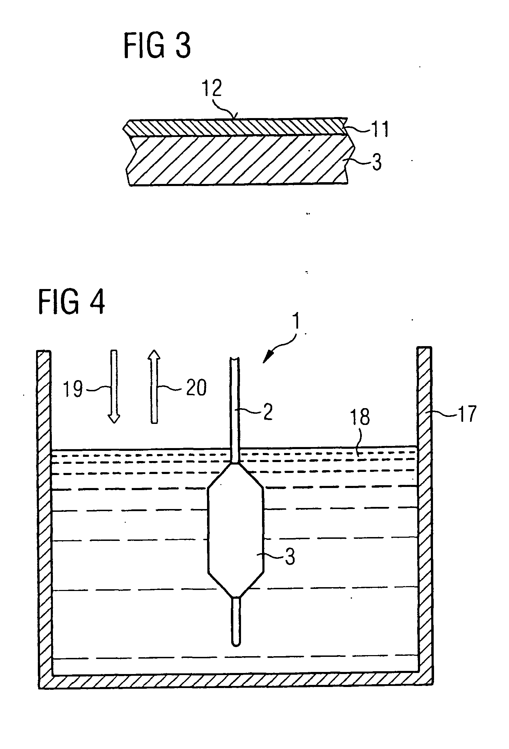

Die durch das Eintauchen in die erste Lösung 8 entstandene Beschichtung auf der Oberfläche des Ballons 3 ist schematisch in der Figur 3 gezeigt. Die Figur 3 zeigt einen Schnitt durch einen Teil der Oberfläche des Ballons 3. Auf der Oberfläche des Ballons 3 befindet sich eine Wirkstoffschicht 11 aus Paclitaxel, die ihrerseits eine lackartige Oberfläche 12 aufweist. Die Paclitaxelschicht 11 ist insgesamt transparent.

Der wie zuvor beschrieben beschichtete Ballon 3 des Ballonkatheters 1 wird in einem optionalen Schritt, der im vorliegenden Ausführungsbeispiel zur Anwendung kommt, in eine weitere, nicht-gesättigte Lösung 18 von Paclitaxel in Methylenchlorid eingetaucht. Der optionale Schritt ist schematisch in der Figur 4 dargestellt. Die Figur 4 zeigt ein Gefäß 17, in welchem sich die nicht-gesättigte Lösung 18 befindet. Die nicht-gesättigte Lösung 18 hat eine geringere Konzentration als die im ersten Schritt verwendete erste Lösung 8. Es kann sich bei der nicht-gesättigten Lösung 18 zum Beispiel um eine Lösung von Paclitaxel in Methylenchlorid mit einer Konzentration von 100 mg/ml handeln. Alternativ dazu sind auch andere Lösungsmittel wie zum Beispiel Chloroform, Ethanol oder Lösungsmittelgemische verwendbar. Das Eintauchen des Ballons 3 in die nicht-gesättigte Lösung 18 ist in der Figur 4 durch einen Pfeil 19 gekennzeichnet. Das anschließende Herausziehen des Ballons 3 aus der nicht-gesättigten Lösung 18 ist durch einen Pfeil 20 gekennzeichnet.

Vorzugsweise wird der Ballon 3 innerhalb von bis zu 3 Minuten nach dem Herausziehen aus der ersten Lösung 8 kurzzeitig, zum Beispiel für ca. 1 Sekunde, in die nicht-gesättigte Lösung 18 eingetaucht. Alternativ dazu kann der Ballon 3 auch lediglich mit der nicht-gesättigten Lösung 18 benetzt werden. Dies kann insbesondere durch Besprühen erfolgen.

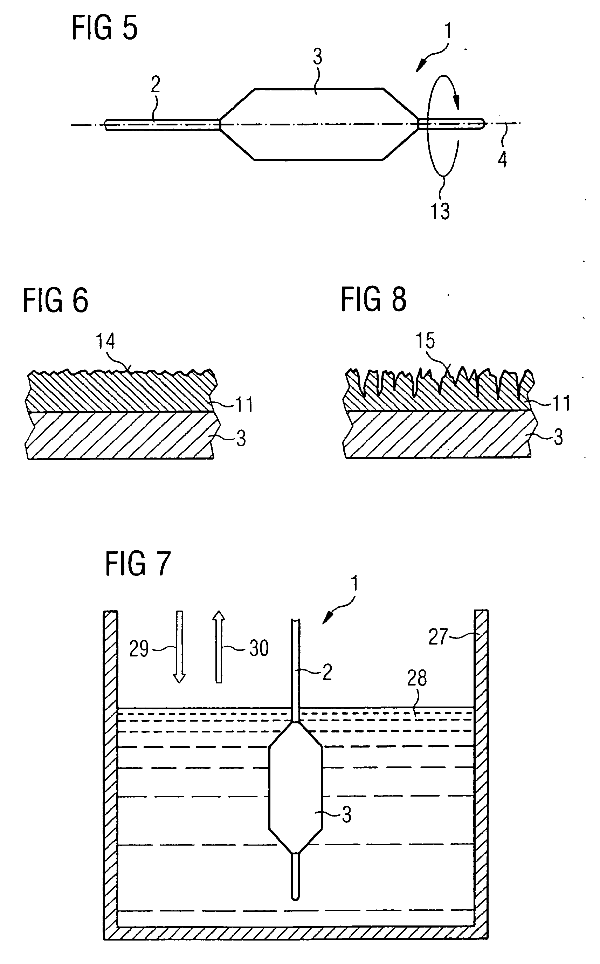

Zur möglichst gleichmäßigen Trocknung des Ballons 3 nach dem Benetzen beziehungsweise Eintauchen in die nicht-gesättigte Lösung 18 wird der Ballonkatheter 1 beziehungsweise der Ballon 3 unmittelbar in eine horizontale Lage in Bezug auf seine Längsachse 4 verbracht und in einem Luftstrom um seine Längsachse 4 gedreht. Dies ist schematisch in der Figur 5 gezeigt. Die Drehung des Ballonkatheters 1 um seine Längsachse 4 ist durch einen Pfeil 13 angedeutet.

Durch das Eintauchen des Ballons 3 in die nicht-gesättigte Lösung 18 beziehungsweise das Benetzen des Ballons 3 mit der nicht-gesättigten Lösung 18 wird die Wirkstoffbeladung auf der Ballonoberfläche, im vorliegenden Beispiel die Paclitaxelbeladung, erhöht. Zudem wird die gesamte Beschichtung spröde beziehungsweise versprödet partiell. Weiterhin wird die gesamte Beschichtung optisch weniger transparent, also milchiger. Insgesamt führt die beschriebene Behandlung mit der nicht-gesättigten Lösung 18 zu einem höheren Wirkstoffabtrag, also zu einem höheren Paclitaxelabtrag, von der Ballonoberfläche in Folge von Reibung.

Die Figur 6 zeigt schematisch einen Schnitt durch einen Teil der Ballonoberfläche 3 nach der Behandlung mit der nicht-gesättigten Lösung 18. Die auf der Oberfläche des Ballons 3 befindliche Wirkstoffschicht 11, bei der es sich im vorliegenden Beispiel um eine Paclitaxelschicht handelt, weist ihrerseits eine spröde, kreideartige Oberfläche 14 auf.

Im Anschluss an die im vorliegenden Ausführungsbeispiel vorgenommene optionale Behandlung mit der nicht-gesättigten Lösung 18 wird der Ballon 3 in eine gesättigte Lösung von beispielsweise Paclitaxel in einem azeotropen Gemisch von beispielsweise Ethanol und Wasser ve.rsetzt mit 0,1 % Essigsäure eingetaucht. Dies ist schematisch in der Figur 7 dargestellt.

Die Figur 7 zeigt ein Gefäß 27, in welchem sich die zweite, gesättigte Lösung des Paclitaxels in einem Gemisch 28 aus Ethanol und Wasser befindet. Es kann sich dabei zum Beispiel um das azeotrope Gemisch aus 96 % Ethanol und 4 % Wasser, bezogen jeweils auf das Gewicht, handeln. Das azeotrope Gemisch kann zudem mit 0,1 % Essigsäure, bezogen auf das Volumen, versetzt sein. Die Essigsäure bewirkt eine Stabilisierung des verwendeten Wirkstoffes, im vorliegenden Beispiel also des Paclitaxels. Die Löslichkeit von Paclitaxel ist in dem azeotropen Gemisch geringer als in der gesättigten Lösung 8 und der nicht-gesättigten Lösung 18 der im Rahmen der vorangegangenen Schritte verwendeten Lösungsmittel.

Vorzugsweise wird der zunächst mit der gesättigten Lösung 8 und mit der nicht-gesättigten Lösung 18 wie zuvor beschrieben behandelte Ballon 3 innerhalb von bis zu 3 Minuten nach dem Herausziehen aus der nicht-gesättigten Lösung 18 kurzzeitig in die zweite, gesättigte Lösung in azeotropem Gemisch 28 eingetaucht oder mit dieser benetzt. Das Eintauchen des Ballons 3 in die zweite, gesättigte Lösung in azeotropem Gemisch 28 ist in der Figur 7 durch die Bezugsziffer 29 gekennzeichnet. Das Herausziehen des Ballons 3 aus der zweiten, gesättigten Lösung in azeotropem Gemisch 28 ist in der Figur 7 durch die Bezugsziffer 30 gekennzeichnet.

Der Ballon 3 wird vorzugsweise für ca. 1 Sekunde in die zweite, gesättigte Lösung in azeotropem Gemisch 28 eingetaucht. Die Dauer des Eintauchens ist abhängig von der nach der Behandlung mit der ersten Lösung 8 erzielten Schichtdicke und kann bis zu 20 Sekunden betragen. Nach dem Herausziehen 30 des Ballons 3 aus der zweiten, gesättigten Lösung in dem azeotropen Gemisch 28 wird der Ballon 3 zur möglichst gleichmäßigen Trocknung unmittelbar in die Horizontale verbracht und in einem Luftstrom um seine Längsachse 4 gedreht, wie im Zusammenhang mit der Figur 5 bereits beschrieben wurde.

In Folge der beschriebenen Behandlung des Ballons 3 mit der zweiten, gesättigten Lösung von Paclitaxel in dem azeotropen Gemisch 28 wird die gesamte Beschichtung 11 spröder und optisch weniger transparent, also milchiger. Dies ist schematisch in der Figur 8 dargestellt. Die Figur 8 zeigt schematisch einen Schnitt durch einen Teil der Oberfläche des Ballons 3 im Anschluss an die Behandlung mit der zweiten, gesättigten Lösung in dem azeotropen Gemisch 28. Auf der Oberfläche des Ballons 3 befindet sich eine Wirkstoffschicht aus Paclitaxel, die ihrerseits eine spröde, kreideartige Oberfläche 15 aufweist. Im Vergleich zu der in der Figur 6 gezeigten Oberfläche 14 der Paclitaxelschicht 11 ist die in Folge der Behandlung mit der zweiten, gesättigten Lösung in einem azeotropen Gemisch 28 entstandene Oberfläche 15 stärker versprödet und weist einen höheren Paclitaxelabtrag bei Reibung auf.

Prinzipiell kann die im Zusammenhang mit den Figuren 4 bis 6 beschriebene Behandlung mit der weiteren, nicht-gesättigten Lösung 18, also das Eintauchen des Ballons 3 in die Lösung 18 beziehungsweise das Benetzen der Oberfläche des Ballons 3 mit der nicht-gesättigten Lösung 18, weggelassen werden. In diesem Fall wird der Ballon 3 innerhalb von maximal 3 Minuten nach dem vollständigen Herausziehen aus der ersten Lösung 8 in die zweite, gesättigte Lösung in dem azeotropen Gemisch 28 eingetaucht beziehungsweise mit dieser benetzt. Wird die Behandlung mit der nicht-gesättigten Lösung 18 weggelassen, ist die Wirkstoffbeladung auf der Oberfläche des Ballons 3 insgesamt geringer. Ebenso ist in diesem Fall die Versprödung der erzeugten Oberfläche der Wirkstoffbeschichtung 11 größer.

Grundsätzlich kann die Oberfläche des Ballons 3 sowohl vollständig als auch nur teilweise beschichtet werden. Insbesondere kann nur der zylinderförmige Bereich 6 des Ballons 3 mit dem Wirkstoff erfindungsgemäß beschichtet werden, da hauptsächlich der zylinderförmige Bereich 6 des Ballons 3 mit dem umliegenden Gewebe im zu behandelnden Körper in Kontakt kommt.

## Patentansprüche

1. Verfahren zum zumindest teilweisen Beschichten der Oberfläche eines Ballons (3) eines Ballonkatheters (1) mit einem Wirkstoff, umfassend die folgenden Schritte:
a) Erzeugen einer Wirkstoffschicht (11) auf der Oberfläche des Ballons (3) durch
aa) Beladung der Oberfläche des Ballons (3) mit dem Wirkstoff über eine zumindest teilweise Benetzung der Oberfläche mit einer ersten Lösung (8), welche ein erstes Lösungsmittel und den Wirkstoff umfasst,
ab) Trocknen der in Schritt aa) erhaltenen benetzten Oberfläche,
b) Verspröden der Wirkstoffschicht mit einer zweiten, gesättigten Lösung des Wirkstoffs, welche weiterhin ein Lösungsmittel, vorzugsweise ein zweites Lösungsmittel umfasst, wobei der mit der ersten Lösung (8) des Wirkstoffs benetzte Teil der Oberfläche mit der zweiten, gesättigten Lösung des Wirkstoffs benetzt wird.

2. Verfahren nach Anspruch 1, wobei die zweite, gesättigte Lösung Ethanol oder Azeton umfasst..

3. Verfahren nach Anspruch 1 oder 2, wobei die zweite, gesättigte Lösung ein azeotropes Lösungsmittel umfasst.

4. Verfahren nach einem der Ansprüche 1 bis 3, wobei die Oberfläche des Ballons (3) mit der ersten Lösung (8) eines Wirkstoffs durch Eintauchen (9) des Ballons (3) in die erste Lösung (8) benetzt wird und der Ballon (3) mit einer Geschwindigkeit von bis zu 10 mm/s aus der ersten Lösung (8) herausgezogen wird.

5. Verfahren nach einem der Ansprüche 1 bis 4, wobei die Oberfläche des Ballons (3) nach dem Benetzen mit der ersten Lösung (8) eines Wirkstoffs und vor dem Benetzen mit der zweiten, gesättigten Lösung mit einer weiteren Lösung (18) des Wirkstoffs benetzt wird.

6. Verfahren nach Anspruch 5, wobei das Benetzen der Oberfläche des Ballons (3) mit der weiteren Lösung (18) des Wirkstoffs durch Eintauchen (19) des Ballons (3) in die weitere Lösung (18), insbesondere für maximal 20 s erfolgt.

7. Verfahren nach Anspruch 5 oder 6, wobei die Oberfläche des Ballons (3) mit der weiteren Lösung (18) des Wirkstoffs maximal 3 min nach dem Benetzen mit der ersten Lösung (8) des Wirkstoffs benetzt wird.

8. Verfahren nach einem der Ansprüche 5 bis 7, wobei die Oberfläche des Ballons (3) nach dem Benetzen mit der weiteren Lösung (18) des Wirkstoffs getrocknet wird.

9. Verfahren nach einem der Ansprüche 1 bis 8, wobei der verwendete Wirkstoff Tretinoin und/oder Tretinoinderivate und/oder Orphanrezeptoragonisten und/oder Elafinderivate und/oder Corticosteroide und/oder Steroidhormone und/oder Paclitaxel und/oder Paclitaxelderivate und/oder Rapamune und/oder Tracrolimus und/oder hydrophobe Proteine und/oder zellproliferationsverändernde Substanzen umfasst.

10. Verfahren nach einem der Ansprüche 1 bis 9, wobei als das erste Lösungsmittel Methylenchlorid, Chloroform, Alkohol, Azeton, Diethylether, flüssige Kohlenwasserstoffe, Toluol, Tetrahydrofuran (THF) oder Essigester verwendet wird.

11. Verfahren nach einem der Ansprüche 1 bis 10, wobei das Lösungsmittel Ethanol und Wasser umfasst.

12. Verfahren nach einem der Ansprüche 1 bis 11, wobei die zweite, gesättigte Lösung Essigsäure umfasst.

13. Ballon eines Ballonkatheters (1), erhältlich durch ein Verfahren nach einem der Ansprüche 1 bis 12.

14. Ballonkatheter, umfassend einen Ballon nach Anspruch 13.

## Claims

1. Method for at least partial coating of the surface of a balloon (3) of a balloon catheter (1) with an active ingredient, comprising the following steps:
a) preparing an active ingredient layer (11) on the surface of the balloon (3) by
aa) applying an active ingredient to the surface of the balloon (3) by an at least partial wetting of the surface with a first solution (8) comprising a first solvent and the active ingredient,
ab) drying the wetted surface obtained by step aa),
b) making brittle the active ingredient layer with a second, saturated solution of the active ingredient, furthermore comprising a solvent, preferably a second solvent, wherein the part of the surface wetted with the first solution (8) of the active ingredient is wetted with the second, saturated solution of the active ingredient.

2. Method according to claim 1, wherein the second, saturated solution comprises ethanol or acetone.

3. Method according to claim 1 or 2, wherein the second, saturated solution comprises an azeotropic solvent.

4. Method according to any of claim 1 to 3, wherein the surface of the balloon (3) is wetted with the first solution (8) of an active ingredient by immersion (9) of the balloon (3) in the first solution (8) and the balloon (3) is removed at a speed of up to 10 mm/s from the first solution.

5. Method according to any of claims 1 to 4, wherein the surface of the balloon (3) after wetting with the first solution (8) of an active ingredient and before wetting with the second, saturated solution, is wetted with an additional solution (18) of the active ingredient.

6. Method according to claim 5, wherein wetting of the surface of the balloon (3) with the additional solution (18) of the active ingredient is realized by immersion (19) of the balloon (3) in the additional solution (18), in particular for at the most 20 s.

7. Method according to claim 5 or 6, wherein the surface of the balloon (3) is wetted with the additional solution (18) of the active ingredient at the latest 3 min. after wetting with the first solution (8) of the active ingredient.

8. Method according to any of claims 5 to 7, wherein the surface of the balloon (3) is dried after wetting with the additional solution (18) of the active ingredient.

9. Method according to any of claims 1 to 8, wherein the employed active ingredient is tretinoin and/or tretinoin derivatives and/or orphan receptor agonists and/or elafin derivatives and/or corticosteroids and/or steroid hormones and/or paclitaxel and/or paclitaxel derivatives and/or rapamune and/or tacrolimus and/or hydrophobic proteins and/or substances that modify cell proliferation.

10. Method according to any of claims 1 to 9, wherein as the first solvent methylene chloride, chloroform, alcohol, acetone, diethyl ether, liquid hydrocarbons, toluene, tetrahydrofurane (THF) or ethyl acetate is used.

11. Method according to any of claims 1 to 10, wherein the solvent comprises ethanol and water.

12. Method according to any of claims 1 to 11, wherein the second, saturated solution comprises acetic acid.

13. Balloon of a balloon catheter (1) obtainable by a method according to any of claims 1 to 12.

14. Balloon catheter comprising a balloon (3) according to claim 13.

## Revendications

1. Procédé pour le revêtement au moins partiel de la surface d'un ballonnet (3) d'un cathéter à ballonnet (1) avec une substance active, comprenant les étapes suivantes:
a) la production d'une couche de substance active (11) sur la surface du ballonnet (3) en
aa) chargeant la surface du ballonnet (3) avec la substance active par un mouillage au moins partiel de la surface avec une première solution (8), qui comprend un premier solvant et la substance active,
ab) séchage de la surface mouillée obtenue à l'étape aa),
b) rendre friable la couche de substance active avec une deuxième solution saturée de la substance active, qui comprend en outre un solvant, de préférence un deuxième solvant, où la partie de la surface mouillée avec la première solution (8) de la substance active est mouillée avec la deuxième solution saturée de la substance active.

2. Procédé selon la revendication 1, où la deuxième solution saturée comprend de l'éthanol ou de l'acétone.

3. Procédé selon la revendication 1 ou 2, où la deuxième solution saturée comprend un solvant azéotrope.

4. Procédé selon l'une des revendications 1 à 3, où la surface du ballonnet (3) est mouillée avec la première solution (8) d'une substance active en plongeant (9) le ballonnet (3) dans la première solution (8), et le ballonnet (3) est retiré à une vitesse allant jusqu'à 10 mm/s de la première solution (8).

5. Procédé selon l'une des revendications 1 à 4, où la surface du ballonnet (3), après le mouillage avec la première solution (8) d'une substance active, et avant le mouillage avec la deuxième solution saturée, est mouillée avec une autre solution (18) de la substance active.

6. Procédé selon la revendication 5, où le mouillage de la surface du ballonnet (3) avec l'autre solution (18) de la substance active a lieu en plongeant (19) le ballonnet (3) dans l'autre solution (18), en particulier pendant 20 s au maximum.

7. Procédé selon la revendication 5 ou 6, où la surface du ballonnet (3) est mouillée avec l'autre solution (18) de la substance active au maximum 3 min après le mouillage avec la première solution (8) de la substance active.

8. Procédé selon l'une des revendications 5 à 7, où la surface du ballonnet (3), après le mouillage avec l'autre solution (18) de la substance active, est séchée.

9. Procédé selon l'une des revendications 1 à 8, où la substance active utilisée comprend de la trétinoïne et/ou des dérivés de trétinoïne et/ou des agonistes récepteurs d'orphan et/ou des dérivés d'élafine et/ou des corticostéroïdes et/ou des hormones stéroïdes et/ou du Paclitaxel et/ou des dérivés de Paclitaxel et/ou des Rapamunes et/ou du tracrolimus et/ou des protéines hydrophobes et/ou des substances modifiant la prolifération des cellules.

10. Procédé selon l'une des revendications 1 à 9, où est utilisé comme premier solvant du chlorure de méthylène, chloroforme, alcool, acétone, diéthyléther, hydrocarbures liquides, toluol, tétrahydrofuranne (THF) ou éther acétique.

11. Procédé selon l'une des revendications 1 à 10, où le solvant comprend de l'éthanol et de l'eau.

12. Procédé selon l'une des revendications 1 à 11, où la deuxième solution saturée comprend de l'acide acétique.

13. Ballonnet d'un cathéter à ballonnet (1), pouvant être obtenu par un procédé selon l'une des revendications 1 à 12.

14. Cathéter à ballonnet, comprenant un ballonnet selon la revendication 13.
